# EUROPEAN PATENT APPLICATION

(11) **EP 3 517 549 A1**
(43) Date of publication of application: **31.07.2019**
(21) Application number: 18153140.1
(22) Date of filing: 24.01.2018
(51) Int. Cl.: C07K 16/28

(54) **ANTIBODY AGAINST ALPHA-11 INTEGRIN AND ITS USE**

(71) Applicant: Bergen Teknologioverføring AS, 5006 Bergen (NO)
(72) Inventor: GULLBERG, Elon Donald, 5006 Bergen (NO); SMELAND, Hilde Ytre-Hauge, 5006 Bergen (NO); AKSLEN, Lars Andreas, 5006 Bergen (NO); REED, Rolf, 5006 Bergen (NO)
(74) Representative: Gramm, Lins & Partner Patent- und Rechtsanwälte PartGmbB

(57) **Abstract**

In a first aspect, the present invention relates to an antibody directed against the alpha-11 integrin subunit, in particular, said antibody is an antibody binding the same epitope as the antibody produced by the hybridoma deposited as DSM ACC3320. Further, the present invention relates to a method for detecting the alpha-11 integrin subunit in a sample. In particular, the antibody according to the present is an antibody suitable for use in samples being cryopreserved and cryosectioned or formaldehyde fixed and paraffin embedded. The present invention relates further to a kit for detecting the alpha-11 integrin subunit containing the antibody according to the present invention. Finally, the present invention provides the antibody in a humanized form. The antibody, in particular, in its humanized forms are useful for antibody drug conjugates.

## Description

In a first aspect, the present invention relates to an antibody directed against the alpha-11 integrin subunit, in particular, said antibody is an antibody binding the same epitope as the antibody produced by the hybridoma deposited as DSM ACC3320. Further, the present invention relates to a method for detecting the alpha-11 integrin subunit in a sample. In particular, the antibody according to the present is an antibody suitable for use in tissue samples being cryopreserved and cryosectioned or formaldehyde fixed and paraffin embedded. The present invention relates further to a kit for detecting the alpha-11 integrin subunit containing the antibody according to the present invention. Finally, the present invention provides the antibody in a humanized form. The antibody, in particular, in its humanized forms are useful for antibody drug conjugates.

### Prior art

Integrins are heterodimeric cell surface receptors composed of non-covalently associated alpha and beta subunits, which act as cell surface links to extracellular matrix (ECM) and to other cells in dynamic cell-cell linkages.

Integrin subunits are composed of different domains with different functions. The extracellular domain of collagen-binding alpha integrin chain contain an inserted alpha I domain, which is responsible for collagen-binding without direct involvement of the beta subunit. Whereas different integrin alpha chains display conserved regions including their cytoplasmic tail, the cytoplasmic tails of integrin alpha chains show little sequence similarity except for the vary proximal membrane sequence GFFXR. Of note, alpha-11 integrin lacks this conserved sequence but contains the GFFRS sequence. This conserved sequence is integral for the activity of the integrin. Namely, mutations of this sequence in general result in integrins being constitutively active.

Integrins are involved in various functions including various signal transduction pathways mediating cellular signals. These signal transduction pathways include the regulation of the cell cycle, the organization of the intracellular cytoskeleton, the movement of new receptors to the cell membrane, etc. The presence of integrins allows rapid and flexible responses to events at the surface. The integrin alpha-11 is a polypeptide that is encoded in humans by the ITGA11 gene. It is described that the integrin alpha-11 binds collagen when present as a heterodimer with beta-1 integrin. Alpha-11 beta-1 integrin represents a collagen receptor, which is the latest identified member of the integrin family. This integrin heterodimer has distinct functions *in vivo* from other collagen-binding integrins. It was demonstrated in mice that collagen-binding integrins are dispensable for normal development, but suggest important roles in tissue remodeling events occurring in wound healing, fibrosis and tumor-stroma interactions.

For example, it is described that non-small lung cancers (NSCLC) express alpha-11 integrin subunit in the activated stroma, when it has the potential to be a biomarker for activated cancer associated fibroblasts [1-3].

The importance of the tumor microenvironment for tumor growth and tumor spread is increasingly being recognized. The major cell types in the tumor stroma of solid tumors include cancer-associated fibroblasts (CAFs) of varying origin, endothelial cells, pericytes, mesenchymal stem cells, tumor stem cells and immune cells. The ECM in addition to serving as a structural scaffold serves as a reservoir of growth factors and cytokines, which take part in bidirectional communication that occurs between the stroma and the tumor cells. For example, CAFs produce collagen cross-linking enzymes of the LOX family, which can increase stiffness of ECM, affecting the growth and invasion of tumor cells. CAFs thus constitute a group of fibroblastic cells of different origin, some of which share characteristics with myofibroblasts in granulation tissue during wound healing and tissue fibrosis. The mesenchymal derived CAF population seems to represent a heterogeneous cell mixture compared to resident tissue fibroblasts in typical resting tissue. For contractile activated myofibroblasts and CAFs the protein alpha smooth muscle actin (alpha SMA) has been suggested as a suitable marker. However, careful analysis of collagen producing activated fibroblast in heart, lung and kidney fibrosis has revealed that only a fraction of these fibroblasts expressed alpha SMA suggesting that alpha SMA is an inconsistent marker of activated collagen producing cells [4]. There is thus a need for better cell type specific markers to be able to understand the dynamics of different CAF population in the tumor stroma. As noted, NSCLC stroma express integrin alpha-11 and, in addition, recent studies suggest that the stroma expression of alpha-11 in NSCLC correlates with LOXL1 expression and tumor stiffness. In fibrosis models importance of integrins have been demonstrated. Although expression of alpha-11 integrin in NSCLC in the activated stroma has been described, and regulation of cancer stroma stiffness of said integrin as well as promoting tumorgenicity and metastases, is discloses, a suitable marker to discriminate between different subclasses of fibroblasts is lacking. This is particularly true for typical tissue samples of tumor patients and the fibrotic tissues in various types of fibrosis These samples obtained as biopsies or obtained from the tumor during surgery, are acetone fixed or formaldehyde fixed before embedding the sample into a suitable embedding medium. The most often used medium for embedding samples is paraffin. However, markers suitable for specific labeling of different fibroblasts subclasses in paraffin embedded samples are rare. In particular, for alpha-11 integrin, no suitable markers are described in the literature so far.

### Brief description of the present invention

Hence, an object of the present invention is the provision of a biomarker suitable for determining alpha-11 integrin subunit in samples, in particular, tissue samples, like tissue samples obtained from cancer and fibrosis patients.

The present inventors aim in providing an antibody suitable for determining the alpha-11 integrin subunit in fixed samples, in particular, in acetone fixed or formaldehyde fixed samples, e.g. in samples being cryopreserved or paraffin embedded.

In a first aspect, the present invention provides an antibody that binds to the same epitope of the alpha-11 integrin subunit as bound by 210 F4B6A4 produced by the hybridoma deposited as DSM ACC3320.

In an embodiment, the antibody is the specific antibody produced by the hybridoma deposited as DSM ACC3320.

In a further aspect, the use of the antibody according to the present invention as a marker for the expression of the alpha-11 integrin subunit, in particular in tissue.

In a further embodiment, the present invention relates to a method for determining the alpha-11 integrin subunit expression in a sample containing cells and/or tissue. The method includes the step of incubating the sample with an antibody according to the present invention and determining binding of said antibody specifically to the alpha-11 integrin subunit by suitable detection means including immunohistological and immunocytochemical detection. The method is particularly suitable for detecting the alpha-11 integrin subunit in cryopreserved or paraffin embedded samples, e.g. tissue samples from cancer and fibrosis patients.

In a further aspect, the present invention provides a kit for detecting alpha-11 integrin subunit in a sample comprising the antibody according to the present invention and detection means for detecting binding specifically of the antibodies according to the present invention to the alpha-11 integrin subunit present in said sample.

Finally, the present invention provides molecules being antibody drug conjugates which are suitable for pharmaceutical purposes. In particular, said antibody drug conjugates contain antibodies being humanized by known methods.

### Brief description of the drawings

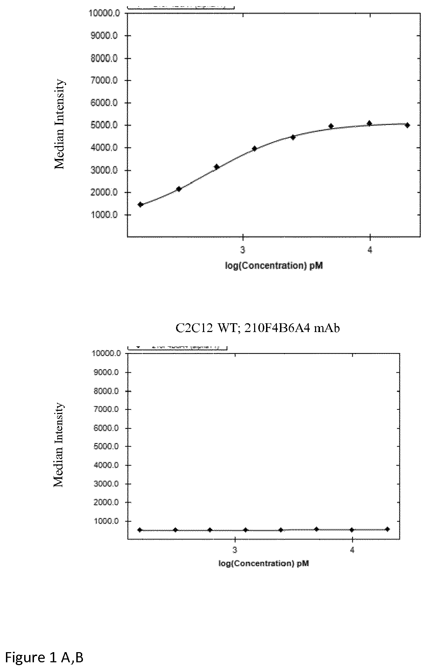

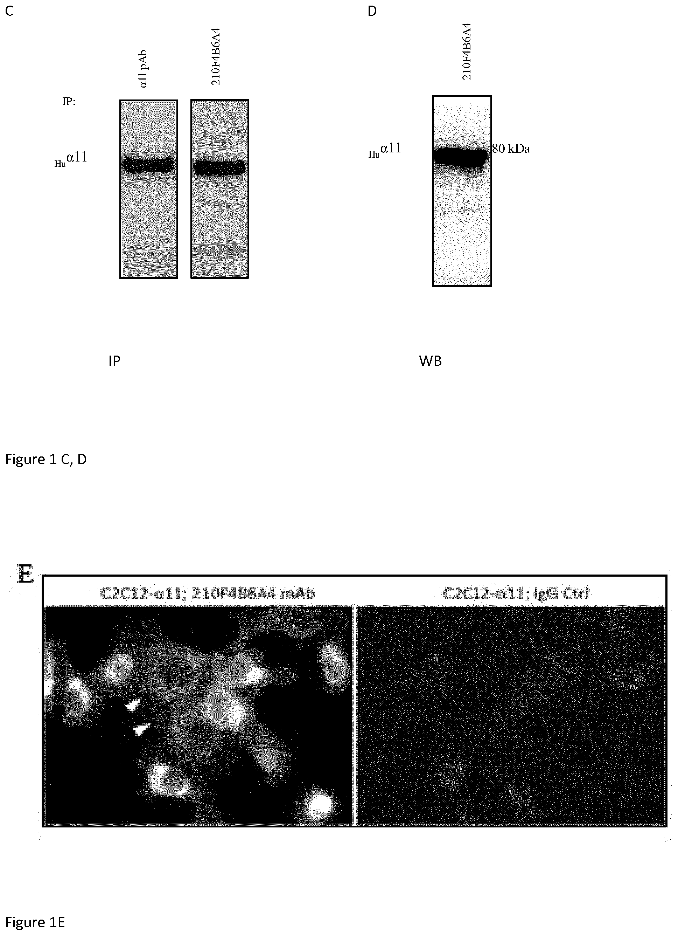

Figure 1. Immunological characterization of 210F4B6A4 mAb
**A-B.** C2C12 mouse myoblast cells expressing human integrin α11-EGFP or WT (wildtype) C2C12 cells were stained with 210F4B6A4 mAb for 1 hour at 4°C. Cells were then washed and stained with goat anti-mouse R-Phycoerythrin IgG. Samples were analysed by flow cytometry using Intellicyt iQue and fluorescence intensity was measured.
**C**. Immunoprecipitation (IP) of integrin α11β1 with the 210F4B6A4 mAb and the polyclonal α11 antibody (α11 pAb)[5]. Protein bands of integrin chain α11 at the expected size after immunoprecipitation using α11 antibodies followed by Western blotting and detection using the polyclonal α11 antibody.
**D.** Western-blotting (WB) using the α11 210F4B6A4 mAb on C2C12-hu a11-EGFP lysate, shown is the protein band of expected size of α11-EGFP (180 kDa).
**E.** C2C12 mouse myoblast cells expressing human integrin α11 were allowed to attach on collagen I-cocated coverslips for 2 hours, cell were then fixed with methanol and incubated with with 210F4B6A4 mAb or control IgG. Bound antibody was detected using goat anti-mouse Alexa Fluor 594. Integrin α11 staining in cell focal adhesions is indicated by white arrowheads.

### Detailed description of the present invention

The present invention aims to provide new antibodies suitable for specifically binding the alpha-11 integrin subunit. Namely, the present invention provides antibodies that's bind to the same epitope of the alpha-11 integrin subunit as bound by 210F4B6A4 antibody produced by the hybridoma deposited as DSM ACC3320. The antibodies according to the present invention are suitable for immune detection of the antigen, namely, the alpha-11 integrin subunit, in samples, in particular, in cryopreserved or paraffin embedded samples.

As used herein, the term "comprise" or "comprising" as well as "contain" or "containing" include the embodiment of "consist" and "consisting of".

Further, the term "antibody" refers to a polypeptide encoded by an immunoglobulin gene or functional fragments thereof that specifically binds and recognizes an antigen. The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta or epsilon, which in turn define the immunoglobulin classes, IgG, IgM, IgA, IgD and IgE, respectively.

The immunoglobulin (antibody) structure unit comprises typically a tetramer composed of two identical pairs of polypeptide chains, each pair having one light and one heavy chain. The N-terminus of each chain defines a variable region of about 100 to 110 or more amino acids primarily responsible for antigen recognition. Thus, the terms "variable heavy chain", V_{H}, or VH, refer to the variable region of an immunoglobulin heavy chain including an Fv, scFv, dsFv or Fab, while the terms "variable light chain", "V_{L}" or "vL" refers to the variable region of the immunoglobulin light chain, including of an Fv, scFv, dsFv or Fab.

Examples of antibody functional fragments include but are not limited to complete antibody molecules, antibody fragments, such as Fv, single chain Fv (scFv), complementarity determining regions (CDRs), VL (light chain variable region), VH (heavy chain variable region), Fab, F(ab)2' and any combination of those or any other functional portion of an immunoglobulin peptide capable of binding to target antigen. The respective antibody and antibody fragments can be obtained by variety of methods known to the skilled person, namely, either by digestion of an intact antibody with an enzyme or by de novo synthesis. The de novo synthesis can either be chemically or by using recombinant DNA technology. Unless otherwise indicated, the term antibody includes antibody fragments obtained by the methods known in the art also including phage display libraries. The term "antibody" also includes bivalent or bispecific molecules, diabodies, triabodies and tetrabodies etc.

The term "humanized" antibody is an antibody that retains the reactivity of a non-human antibody while being less immunogenic in humans. This can be achieved by methods along known in the art by retaining the non-human CDR regions and replacing the remaining parts of the antibodies with the human counterparts, a technic well known to the skilled person.

"Single chain Fv (scFv)" or "single chain antibodies" refers to a protein wherein the V_{H} and the V_{L} regions of a scFv antibody comprise a single chain which is folded to create an antigen binding site similar to that found in two chain antibodies. Methods for producing the same are described in the art. Single chain Fv antibodies optionally include a peptide linker of no more than 50 amino acids in length.

The phrase "specifically (or selectively) binds to an antibody" when referring to a protein or peptide, refers to a binding reaction which is determinative of the presence of the protein in the presence of a heterogeneous population of proteins and other biologics. That is, in the designated immunoassay conditions typically present in said immunoassays, the specific antibodies bind to a particular protein, in the present case, the antibody according to the present invention to the alpha-11 integrin subunit and do not bind in a significant amount to other proteins present in the sample. Typically, a specific or selective reaction will be at least twice the background signal or noise and more typically more than 10 to 100 times over the background.

The antibody which is in a one embodiment a monoclonal antibody according to the present invention can be obtained from any animal or the human being, whereby the monoclonal antibody from a mouse are preferred. Further, the monoclonal antibody may be altered biochemically, by genetic manipulation, or it may be synthetic, with the antibody possibly lacking portions completely or in parts, said portions being necessary for the recognition of alpha-11 integrin subunit and being substituted by others imparting further advantageous properties to the antibody.

A hybridoma cell line producing a preferred antibody of the present invention, namely, the monoclonal mouse antibody F210F4B6A4, was deposited at the Deutsche Sammlung von Mikroorganismen und Zellkulturen GmbH (DSMZ) in Braunschweig under the number DSM ACC3320 on March 21, 2017.

The term "epitope" also known as "antigenic determinant" is a part of an antigen that is recognized by the immune system in the present context by antibodies. The epitopes according to the present invention may be conformational epitopes and linear epitopes, based on their structure and interaction with the paratope, namely the part of the antibody that binds to the epitope.

The skilled person is well aware of suitable methods to determine whether an antibody binds to the same epitope of the alpha-11 integrin subunit as bound by 210 F4B6A4 produced by the hybridoma deposited as DSM ACC3320. For example, appropriate tests may include experiments were competitive binding assays of the antibody produced by the hybridoma identified herein and the antibody in question are conducted.

An agent that specifically competes for binding reduces the specific binding of an antibody to a polypeptide. That is, competitive binding assays may be conducted to determine appropriate antibodies using the 210F4B6A4 antibody produced by the hybridoma cell line disclosed herewith.

The term "sample" is intended to cover all types of samples suitable for the purpose of the invention. Examples of such samples are serum, sputum, urine, liquor, tissue, and biopsies. In particular, the sample may be a blood sample or a tissue sample. In an embodiment, the tissue sample is a paraffin embedded tissue sample, a cryopreserved sample or a cell sample.

In an embodiment of the present invention, the antibody is the antibody 210F4B6A4 produced by the hybridoma deposited as DSM ACC3320.

In another embodiment, the antibody, which is typically a monoclonal antibody, is an antibody comprising the heavy and light chain complementary determining regions (CDR) of the antibody according to the present invention, in particular, wherein the CDR are the CDR of the monoclonal antibody produced by the hybridoma deposited as DSM ACC3320.

In an another embodiment, the present invention relates to antibodies, in particular, monoclonal antibodies being selected from the group consisting of scFv, Fab, (Fab')₂.

In an embodiment, the antibody according to the present invention, in particular, monoclonal antibodies like the 210F4B6A4 monoclonal antibody or derivatives thereof are useful as a marker of the expression of the alpha-11 integrin subunit in a sample. As noted, the sample may be a body fluid or a tissue sample. In an embodiment of the present invention, the detection of the antibody binding specifically to the alpha-11 subunit according to the present invention is by an immunohistochemical or immunocytochemical method.

The antibodies according to the present invention are excellently suitable in the application of diagnostic problems, e. g. determined by immunohistochemistry with quantitative expression parameters. Alternatively, said antibodies may be used in Western Blot or immunoprecipitation analysis.

For example, the antibody according to the present invention is particularly useful for determining the alpha-11 integrin subunit in samples whereby said samples were fixed before by known methods, for example which wherein the samples are acetone-fixed or formaldehyde-fixed before. That is, the use is particularly for staining alpha-11 integrin subunit in tissues, preferably on cryopreserved or routinely fixed and paraffin embedded tissues. For this the antibodies according to the present inventions may be labelled appropriately, as described above, or employed in combination with label antibodies direct against them or other reagents.

That is, the antibodies according to the present invention can be used diagnostically to monitor protein levels of the alpha-11 integrin subunit in tissue as part of clinical examination or clinical testing procedures, e.g. to determine the efficacy of a given treatment regimen or determining cancer as discussed below. Detection can be facilitated by coupling, e.g. physically linking, the antibody to a detectable substance. Examples of detectable substances include various enzymes, prosthetic groups, fluorescent materials, luminescent materials, bioluminescent materials and radioactive materials. Examples of suitable enzymes include horseradish peroxidase; alkaline phosphatase (-galactosidase, or acetylcholinesterase); examples of suitable prosthetic group complexes include streptavidin/biotin and avidin/biotin; examples of suitable fluorescent materials include fluorescein, fluorescein isothiocyanate, rhodamine, Cy-dyes, alexa fluor-dyes or brilliant violet dyes. Suitable radioactive materials include ¹²⁵I, ¹³¹I, ³⁵S or ³H while luminescent and bioluminescent materials include luminol as well as luciferase, luciferin and aequorin.

As noted above, detection may be also in combination with labelled secondary antibodies directed against the antibody according to the present invention. The skilled person is well known of suitable secondary antibodies which means that these antibodies are directed against the species from which the antibody according to the present invention is derived. Suitable examples in case of mouse monoclonal antibodies include gold anti mouse antibodies labelled with enzymes or dyes etc. which are commercially available.

The antibody are useful for detecting the alpha-11 integrin subunit expression by cultured cells or in cryopreserved tissue or tissues present in paraffin embedded samples. The present antibodies allow to detect routinely and specifically the alpha-11 integrin subunit in cryosections or paraffin embedded samples.

In a further embodiment, the antibody according to the present invention, i.e. the monoclonal antibody according to the present invention, are humanized antibodies. That is, the antibody is a humanized antibody, i.e. an antibody that retains the reactivity of a non-human antibody while being less immunogenic in humans. This can be achieved for instance by retaining the non-human CDR regions and replacing the remaining parts of the antibody with the human counterparts. This is a well established procedure known in the art. In cases were the transfer of the CDR to a human framework leads to a loss of specificity for humanized antibody, back mutations can be introduced to the framework regions of the human portion of the antibodies. These methods are described in the art accordingly.

The antibody according to the present invention, in particular, the humanized antibodies, may be also useful as active agents in pharmaceuticals. That is, these active agents may be used to treat diseases, disorders or conditions where the alpha-11 integrin subunit expression is altered or when alteration of the alpha-11 integrin subunit expression is beneficial to the subject receiving said treatment. These diseases, disorders and conditions include cancer, fibrosis, wound healing and degenerative inflammatory and non-inflammatory diseases such as rheumatoid arthritis and osteoarthritis.

Another embodiment of the present invention relates to a method for determining the alpha-11 integrin subunit expression in a sample whereby said sample comprises cells and tissue, including the step of incubating the sample with the antibody according to the present invention and determining specific binding of said antibody by suitable means including immunohistological and immunohistochemical detection. The detection means are described above including label and marker. Said label and marker may be present with the antibody itself or may be present on secondary antibodies or secondary detection compounds containing a label or marker for detection accordingly.

In an embodiment of the method according to the present invention, the sample are tissue sections, in particular, human tissue sections. Further, in an embodiment of the present invention, the tissue sections are aceton, methanol or aldehyde fixed tissue sections, in particular, said tissue sections, or samples in general, are cryosections or paraffin embedded samples including paraffin embedded tissue sections or paraffin embedded cell sections.

The method according to the present invention may comprise further suitable steps for embedding the tissue and/or cells including paraffin embedding steps. The skilled person is well aware of the steps required for embedding the samples accordingly. In addition, the method according to the present invention may further comprise the necessary steps to treat the paraffin embedded samples to allow detection of antigens by antibodies accordingly.

Moreover, this method according to the present invention for detecting alpha-11 integrin subunit contain further steps of detection including immunohistochemical and immunohistological detection steps, like binding of secondary antibodies and staining of the sections when using enzyme based detection systems or microscopy etc. when using fluorescence based or chromophore based systems.

The use of an method according to the present invention are useful for detecting various types of cancer. In particular, the antibodies according to the present invention as well as the method according to the present invention allow to identify fibroblastoid expression in keratin-negative, vimentin-positive, non-vessel associated stroma of invasive breast carcinoma, ovary adenocarcinoma, skin carcinoma and pancreas adenocarcinoma. In general, the antibodies according to the present invention may serve as a biomarker for CAFs in carcinoma-type tumor characterized by a desmoplastic stroma.

In a further aspect, the present invention relates to a kit for detecting alpha-11 integrin subunit in a sample comprising the antibody according to the present invention. That is, the test kit also named diagnostic kit according to the present invention, in particular for use in the method according to the present invention, is an immunological kit for use in detecting the antibodies according to the present invention bound to the alpha-11 integrin subunit. This kit can generally comprise the antibody according to the present invention. Further, the kits will comprise in suitable container(s), one or more detecting agents, in particular, when the antibodies according to the present invention do not contain a marker or label. If required, the kit further comprises substrate and further means for allowing reaction with an enzyme used as a label for the detecting agent which may be an antibody. The immunodetection agents of the kit can include detectable labels that are associated with or linked to the given detecting agent, typically, a detecting antibody being a secondary antibody. Detectable labels and markers include the labels or markers identified above. Optionally, the kits further comprise positive and negative controls accordingly. The term "determining" or "analyzing" as used herein refers to assessing the presence, absence, level or amount of the specific binding of the antibody to the alpha-11 subunit integrin within the sample.

In an embodiment of the present invention, the presence of the antibodies can be determined using secondary antibodies from different species in the sample and the antibody according to the present invention. In a further embodiment, the kit contain further marker molecules allowing detection of the antibodies bound to the alpha-11 subunit present in the sample by immunohistochemical means including immunofluorescence and immunohistochemistry.

### Examples

The following examples have been included to illustrate modes of the present disclosed subject matter. In light of the present disclosure and the general level of the skilled in the art, those of skilled in the art will appreciate that the following examples are intended to be exemplary only and that numerus changes and modifications can be applied without departing from the scope of the present disclosed subject matter.

### Material and Methods

**Cell lines** - C2C12 cells stably expressing human α11 integrin or human α2 integrin subunits (C2C12-α11 and C2C12-α2, respectively [6]) were cultured in DMEM medium and 10% fetal bovine serum (FBS; Gibco) supplemented with antibiotics.

### Generation of Mab specific to integrin α11 chain

Mabs were produced using established procedures. NT-HRM mice (nanoTools Antikoerpertechnik, Germany) were immunized with human α11β1 integrin (R&D Systems), boosted twice and cell fusion was performed on day 68. Luminex beads coated with α11β1 integrin were used to screen α11 binders. Supernatants from positive clones were tested in FACS using C2C12- α11 cells as positive control and wildtype C2C12 (do not express human integrins), C2C12 -α2 cells and A431 cells (do express human integrin β1, but not α11) as negative controls. Positive clones were further characterized and finally subcloned by limited dilution.

### Immunostaining

Cells were washed in PBS and fixed with ice cold methanol 10 min at -20C. Cells were blocked with 5% BSA/PBS containing 0.1% Triton X-100 for 1 hour at RT. Next, cover slips were incubated with primary antibody, 210F4B6A4, in 5% BSA/PBS with 0.1% Triton X-100 for 1 hour at 37°C. Cells were then washed with 0.05% Tween-20/PBS and incubated with Alexa fluor® 594 goat anti-mouse IgG (1:400, Jackson ImmunoResearch) for 1 hour at RT. Later, coverslips were incubated with DAPI (0.25 µg/ml, Invitrogen) and mounted with ProLong Diamond Antifade mounting medium (Thermo Scientific). Cells were visualized under a Zeiss Axioscope fluorescence microscope and pictures were acquired with a digital AxioCam MRm camera.

### Flow cytometry

Cells were harvested in PBS (without Ca²⁺ and Mg²⁺) and accutase® (Biochrom) and washed in PBS (without Ca²⁺ and Mg²⁺). 40µl of integrin α11 antibody supernatants (diluted 1:8 with Fluorescence activated cell sorting (FACS) buffer (PBS/0.2 mM Ca²⁺, 0.1% BSA and 0.1% Poloxamer 188)) were mixed with 10⁵ cells and incubated 1hour at 4°C. Cells were then washed once with FACS buffer and incubated 1 hour at 4°C with 50µl of PE labelled goat anti-mouse (diluted 1:400 in FACS buffer). Cells were washed once with FACS buffer and the samples analyzed by flow cytometry using Intellicyt iQue.

### Immunoprecipitation

Subconfluent C2C12-α11 cells were lysed using ice-cold solubilization buffer (1% Triton X-100, 0.15 M NaCl, 10 mM Tris-HCI pH 7.4, 1 mM MgCl₂, 1 mM CaCl₂) containing a protease inhibitor cocktail (complete mini EDTA-free, Roche Diagnostics). Cell lysates were centrifuged for 10 min at 4°C at 13000 rpm. The supernatant was pre-cleared by incubation with 100 µg/ml of preimmune IgG and protein A-Sepharose CL 4B (GE Healthcare) at 4°C overnight. In the next step, samples were incubated with 100 µg/ml of rabbit polyclonal anti-human α11 antibody [5] or mouse Mab 210 F4B6A4 hybridoma supernatant. The precipitates were washed three times with high salt buffer (1% Triton X-100, 0.5 M NaCl, 10 mM Tris-HCI pH 7.4, 1 mM MgCl₂, 1 mM CaCl₂) and three times in a physiological salt buffer (0.1% Triton X-100, 0.15 M NaCl, 10 mM Tris-HCI pH 7.4, 1 mM MgCl₂, 1 mM CaCl₂) before solubilization in sodium dodecyl sulphate (SDS) -sample buffer with reducing agents. Proteins were separated by 6% SDS - poly acrylamide gel electrophoresis (PAGE) and proteins were then transferred using iBlot® system. Membranes were blocked with 5% non-fat dry milk (Marvel, UK) in Tris-buffered saline containing 0.1% Tween20 (TBS-T), incubated with primary rabbit polyclonal anti-human α11 antibody or mouse mab 203E3 anti-human integrin α11 supernatant and to β-actin, overnight at 4°C. Following incubations, membranes were washed in TBS-T three times for 10 min, and incubated with goat anti-mouse or goat anti-rabbit HRP conjugated secondary antibodies for 1 h at room temperature. Membranes were developed using ECL™ western blotting systems kit (GE Healthcare), and photographed using the ChemiDoc XRS device and the Quantity One 1-D Analysis Software (Bio-Rad).

It is demonstrated herein, that the antibody according to the present invention is suitable in staining specifically alpha 11 integrin subunit in tissues and cells. In particular, the antibody allow the bind and stain specifically the alpha 11 subunit in tissue, either cryopreserved or paraffin embedded. In particular, in the tumor context, alpha 11 has the potential to serve as a biomarker for tumor tissue characterized by a desmoplastic stroma.

### References

1. Zhu, C.Q., et al., Proc Natl Acad Sci USA, 2007. 104(28): p. 11754-9.
2. Navab, R., et al., Oncogene, 2016. 35(15): p. 1899-908.
3. Parajuli, H., et al., J Oral Pathol Med, 2017. 46(4): p. 267-275.
4. Sun KH, C.Y., Reed NI, Sheppard D, Am J Physiol Lung Cell Mol Physiol., 2016. doi: 10.1152/ajplung.00350.2015**.**
5. Veiling, T., et al., J Biol Chem, 1999. 274(36): p. 25735-42.
6. Tiger, C.F., et al., Dev Biol, 2001. 237(1): p. 116-29.

## Claims

1. An antibody that binds to the same epitope of the alpha-11 integrin subunit as bound by 210 F4B6A4 produced by the hybridoma deposited as DSM ACC3320.

2. The antibody of claim 1 wherein the antibody is 210 F4B6A4 produced by the hybridoma deposited as DSM ACC3320.

3. The antibody of claim 1 or 2 comprising the heavy and light chain complementarity determining regions (CDR) of the antibody as defined in claim 1 or 2, in particular, wherein the CDR are CDR of the antibody produced by the hybridoma deposited as DSM ACC3320.

4. The antibody according to any one of the preceding claims wherein the antibody is selected from the group consisting of scFv, a Fab, and a (Fab')₂.

5. The use of an antibody according to any one of the preceding claims as a marker for the expression of the alpha-11 integrin subunit.

6. The use of an antibody according to claim 5 for detecting the alpha-11 integrin subunit expression in a sample by an immunohistochemical or immunocytochemical method.

7. The use of an antibody according to claim 5 or 6 wherein the sample is a sample which was fixed before, preferably which was acetone-, methanol- fixed or formaldehyde fixed before.

8. The use of antibody according to claim 7 wherein the cells or tissue present in the sample are cryopreserved and cryosectioned or paraffin embedded samples.

9. A method for determining the alpha-11 integrin subunit expression in a sample comprising cells and/or tissue, including the step of incubating the sample with an antibody according to any one of claims 1 to 4 and determining binding of said antibody by suitable means including immunohistological and immunocytochemical detection.

10. The method according to claim 9 wherein the sample are tissue sections, in particular, human tissue sections.

11. The method according to claim 10 wherein the tissue sections are acetone-methanol- or aldehyde fixed tissue sections, in particular, cryosections or paraffin embedded tissue sections.

12. A kit for detecting alpha-11 integrin subunit in a sample comprising an antibody as defined in any one of claims 1 to 4

13. The kit according to claim 12 further comprising means for determining binding of the antibodies.

14. The kit according to claim 13 wherein the means for determining the presence of the antibodies include secondary antibodies from different species than the sample and the antibody as defined in any one of claims 1 to 4.

15. The kit according to any one of claims 12 to 14 further containing marker molecules allowing detection of the antibodies bound to the alpha-11 integrin subunit present in the sample by immunohistochemical means including immunofluorescence.
